**Europäisches Patentamt**

(19) **European Patent Office**

**Office européen des brevets**

(11) Veröffentlichungsnummer: **0 239 648**

**A1**

(12) # EUROPÄISCHE PATENTANMELDUNG

Veröffentlicht nach Art. 158 Abs. 3 EPÜ

(21) Anmeldenummer: **86906503.7**

(22) Anmeldetag: **16.09.86**

Daten der zugrundeliegenden internationalen Anmeldung:

(86) Internationale Anmeldenummer:
PCT/SU86/00083

(87) Internationale Veröffentlichungsnummer:
WO87/02056 (09.04.87 87/08)

(51) Int. Cl.³: **C 12 N 5/00**

(30) Priorität: 03.10.85 SU 3958774

(43) Veröffentlichungstag der Anmeldung:
07.10.87 Patentblatt 87/41

(84) Benannte Vertragsstaaten:
CH DE FR GB LI NL SE

(71) Anmelder: **NAUCHNO-PROIZVODSTVENNOE
OBIEDINENIE 'BIOLAR'**
Latviiskaya SSR
Olaine, 229014(SU)

(71) Anmelder: **INSTITUT POLIOMIELITA I VIRUSNYKH
ENTSEFALITOV AKAD. MED. NAUK SSSR**
Leninsky raion
Moskovskaya obl., 142782, p/o Inst. poliomelita(SU)

(72) Erfinder: **GRACHEV, Viktor Pavlovich** Leninsky raion
Moskovskaya obl.
142782, p/o Institut poliomielita(SU)

(72) Erfinder: **GUTMANIS, Andris Ekabovich**
ul. Lachplesha, 35-24
Riga, 226011(SU)

(72) Erfinder: **ZAVALNY, Mikhail Alexandrovich**
ul. Festivalnaya, 14-6-965
Moscow, 123098(SU)

(72) Erfinder: **ZITSMANIS, Andris Khugovich**
ul. Lokomotives, 44-23
Riga, 226057(SU)

(72) Erfinder: **KLYAVINSH, Maris Karlovich**
ul. Launkalnes, 12-33
Riga, 226036(SU)

(72) Erfinder: **POPOVA, Valentina Dmitrievna**
bulvar Generala Karbysheva, 9-1-45
Mosocw, 142782(SU)

(74) Vertreter: **von Füner, Alexander, Dr. et al,**
Patentanwälte v. Füner, Ebbinghaus, Finck Mariahilfplatz
2 & 3
D-8000 München 90(DE)

(54) **HERSTELLUNGSVERFAHREN FÜR MIKROTRÄGER ZUR ZUCHT VON ZELLEN UND SO ERHALTENE MIKROTRÄGER.**

(57) Verfahren zur Herstellung von Mikroträgern zum Kultivieren von Zellen besteht darin, daß die Herausbildung von Teilchen aus Gelatine mittels Behandlung der Gelatinelösung mit einem Quervernetzungsmittel mit anschließender Zerkleinerung des entstandenen Gelatine-Blockpolymeres bis zur Erzielung der Teilchen unregelmäßiger Form und Abscheidung der Teilchen mit Abmessungen, die das optimale Wachstum der Zellen auf denselben bewirken, erfolgt beziehungsweise die Herausbildung von Teilchen aus Gelatine mittels Zerkleinerung der Trockengelatine bis zur Erzielung der Teilchen unregelmäßiger Form, der Abscheidung der Teilchen mit den Abmessungen, die das optimale Wachstum der Zellen auf denselben bewirken, mit ihrer anschließenden Behandlung mit dem Quervernetzungsmittel erfolgt, und daß nach der Herausbildung der Teilchen diese mit einem prot-eolytischen Ferment behandelt und stabilisiert werden.

Mikroträger zum Kultivieren von Zellen stellen Teilchen aus einem Gelatine-Blockpolymer unregelmäßiger Form mit Abmessungen von 75 bis 350 µm und einer Dichte von 1,03 bis 1,15 g/cm³ dar.

VERFAHREN ZUR HERSTELLUNG VON MIKROTRÄGERN ZUM 0239648
KULTIVIEREN VON ZELLEN UND DIE IN DIESEM VER-
FAHREN ERHALTENEN MIKROTRÄGER

## Gebiet der Technik

Die vorliegende Erfindung bezieht sich auf die mikrobiologische Industrie und betrifft insbesondere ein Verfahren zur Herstellung von Mikroträgern zum Kultivieren
von Zellen sowie die in diesem Verfahren erhaltenen Mikroträger.

## Vorhergehender Stand der Technik

Gegenwärtig zeugen die umfassenden experimentellen
und praktischen Erfahrungen, die in der Welt auf dem Gebiet der großdimensionalen Kultivierung von Zellen gesammelt wurden, von den Vorzügen und Aussichten der Methode
zum Pseudosuspensionskultivieren von oberflächenabhängigen
Zellen an Mikroträgern. Das Prinzip dieser Methode besteht
darin, daß sich die Zellen an den Kleinstteilchen, den in
einem Nährboden suspendierten Mikroträgern befestigen, ausbreiten und wachsen.

An Mikroträgern ist es zur Zeit möglich, folgende Zellen zu züchten: Nierenzellen von Grünaffen, primäre und sekundäre Kulturen: Zellen von Hühnerembryonen, primäre und
sekundäre Kulturen und andere.

Bekannt ist ein Verfahren zur Herstellung von Mikroträgern zum Kultivieren von Zellen, bestehend im Suspendieren
einer Gelatinelösung zwecks Herstellung von aufgeschwemmten
sphärischen Gelatineteilchen, die man durch ein Sieb mit
einer Maschengröße von 100 $\mu$m unter energischem Vermischen
in einem Toluol-Chloroform-Gemisch in Gegenwart eines oberflächenaktiven Stoffes durchsiebt. Nach dem Durchwaschen
mit Azeton und Verdampfung bis zur Trockne der abgeschiedenen Gelatineteilchen mit Abmessungen nicht über 100 $\mu$m
werden diese in Wasser zwecks Quellung suspendiert, dann
mit Glutaraldehyd behandelt, wonach man sie wiederholt durch
das Sieb durchsiebt, das Maschenabmessungen von 100 $\mu$m
aufweist. Die dadurch abgeschiedenen Teilchen werden suspendiert und in einem Autoklaven zwecks Sterilisation dieser
Teilchen bearbeitet. Die erzeugten Probestücke werden zunächst durch ein Sieb mit Maschenabmessungen von 250 $\mu$m

- 2 -

und dann werden die abgeschiedenen Teilchen durch ein Sieb mit Maschengröße von 100 $\mu$m erneut durchsiebt und wiederholt im Autoklaven bearbeitet (PCT-Anmeldung W 82/ 0060, veröffentlicht am 04.03.82).

Die Kompliziertheit der Ausführung dieses Verfahrens ist darauf zurückzuführen, daß es notwendig ist, die Gelatinelösung in speziell dafür ausgerüsteten Reaktoren zu suspendieren und dabei eine komplizierte Zusammensetzung des Gemisches (Dreikomponentengemisch) einzusetzen. Neben dem Gesagten sieht das bekannte Verfahren eine mehrstufige anschließende Behandlung des herzustellenden Produktes, den Einsatz von brandgefährlichen und toxischen Lösungsmitteln sowie die Notwendigkeit vor, eine große Menge von Hauptreaktionsteilnehmer, Glutaraldehyd zu verwenden.

Bekannt ist ebenfalls ein Verfahren zur Herstellung von Mikroträgern zum Kultivieren von Zellen, das besteht, daß man eine Gelatinelösung unter intensivem Vermischen in einem Gemisch suspendiert, das sich aus einem unpolaren organischen Lösungsmittel zusammensetzt, in das man während des Suspendierens ein Quervernetzungsmittel, Dialdehyd einführt.

Die dabei erzeugten sphärischen Teilchen, Mikroträger werden durch ein Sieb durchgesiebt, mit einem organischen Lösungsmittel und dann mit heißem Wasser in Gegenwart von oberflächenaktiven Stoffen ausgewaschen. Dann behandelt man die erzeugten Mikroträger mit einem reduzierenden Reagens, beispielsweise mit einer 10%igen Lösung des Wasserstoffperoxids, zur Reduktion der entstandenen Schiffschen Basen mit dem Ziel der Stabilisierung beim Betrieb und bei der Lagerung sowie zwecks Entfärbung.

Nach dem anschließenden Auswaschen erfolgt die Abscheidung von Teilchen mit Abmessungen von 100 $\mu$m bis 250 $\mu$m (SU-Urheberschein Nr. 1161548, veröffentlich am 15.06.85).

Kennzeichnend für das genannte Verfahren ist die Kompliziertheit seiner Ausführung, die auf die Notwendigkeit zurückzuführen ist, einen speziell dafür ausgerüsteten Reaktor zum Suspendieren zur Verfügung zu haben und eine Mehrkomponenten-Zusammensetzung des Reaktionsmediums zu verwenden. Bei der Anwendung des genannten Verfahrens besteht die Kompliziertheit in dem Auswaschen der erzeugten Mikroträger vom Dispergierungsmittel.

Die bekannten Verfahren ermöglichen es außerdem, Mikroträger sphärischer Form zu erzeugen, die in der Stufe der Kultivierung von Zellen nicht optimal ist, das heißt, sie erschwert das Befestigen der Zellen an die Oberfläche der Mikroträger.

## Offenbarung der Erfindung

Der vorliegenden Erfindung liegt die Aufgabe zugrunde, durch Veränderung der technologischen Arbeitsgänge die Technologie der Herstellung von Mikroträgern zu vereinfachen und ihre Qualität zu verbessern, die ein effektives Wachstum der Zellen bewirkt.

Diese Aufgabe wird dadurch gelöst, daß man im Verfahren zur Herstellung von Mikroträgern zum Kultivieren von Zellen, das die Herausbildung von Teilchen aus Gelatine, die mit einem Quervernetzungsmittel bearbeitet wird, und die Stabilisierung des hergestellten Endproduktes vorsieht, erfindungsgemäß, die Herausbildung der Teilchen aus Gelatine durch Behandlung der Gelatinelösung mit dem Quervernetzungsmittel mit anschließender Zerkleinerung des entstandenen Gelatine-Blockpolymers bis zur Erzielung der Teilchen unregelmäßiger Form und mittels Abscheidung der Teilchen mit Abmessungen, die das optimale Zellenwachstum an denselben gewährleisten, durchführt, beziehungsweise die Herausbildung der Teilchen aus Gelatine durch Zerkleinerung von Trockengelatine bis zur Erzielung der Teilchen unregelmäßiger Form, mittels Abscheidung von Teilchen mit Abmessungen, die das optimale Wachstum der Zellen an denselben gewährleisten, und mittels ihrer anschließenden Bearbeitung mit dem Quervernetzungsmittel erfolgt man nach der Herausbildung der Teilchen vor ihrer Stabilisierung zwecks Schaffung ihrer glatten Oberfläche die Behandlung dieser Teilchen mit einem proteolytischen Ferment durchführt.

Durch das erfindungsgemäße Verfahren wurde es möglich, ihrem Reinheitsgrad nach hochqualitativere Mikroträger zu erzeugen, das heißt, daß in den zu erzeugenden Mikroträgern Beimenungen des Suspendierungsmediums fehlen, deren Vorhandensein früher toxische Einwirkung auf die zu züchtenden Zellen ausübte. Dabei wurde die Technologie der Herstellung durch Reduzierung einer Reihe von Stufen bedeutend vereinfacht. Es entfiel außerdem die Notwendigkeit, komplizierte Apparaturen zum Suspendieren einzusetzen.

Erfindungsgemäß, ist es zweckmäßig, als Quervernetzungsmittel Dialdehyde beziehungsweise Formaldehyd zu verwenden.

Zur Verbesserung der Qualität der Mikroträger und zur Erhöhung ihrer Stabilität bei Lagerung ist es zweckmäßig, erfindungsgemäß, bei der Verwendung des Dialdehyds als Quervernetzungsmittel die Stabilisierung der herausgebildeten Teilchen mittels ihrer wiederholten Behandlung mit dem Dialdehyd und durch anschließende Behandlung dieser Teilchen mit reduzierenden Reagenzien durchzuführen.

Erfindungsgemäß, ist es zweckmäßig, als reduzierendes Reagens Borhydride von Alkalimetallen beziehungsweise Wasserstoffperoxid zu verwenden.

Eine Variante der Ausführung der vorliegenden Erfindung besteht darin, daß bei der Verwendung des Formaldehyds als Quervernetzungsmittel die Stabilisierung der herausgebildeten Teilchen mittels ihrer wiederholten Behandlung mit Formaldehyd erfolgt, was es ermöglicht, die Herstellung des Endproduktes unter Beibehaltung der erreichten hohen Kenndaten seiner Qualität zu verbilligen.

Erfindungsgemäß, ist es zweckmäßig, als proteolytisches Ferment Trypsin beziehungsweise Kollagenase zu verwenden.

Als Erfindung treten auch Mikroträger zum Kultivieren von Zellen auf, die gemäß dem angemeldeten Verfahren hergestellt werden und Teilchen aus einem Gelatine-Blockpolymeres unregelmäßiger Form mit Abmessungen von 75 bis 350 $\mu$m und einer Dichte von 1,03 bis 1,15 g/cm$^3$ darstellen.

Die erfindungsgemäße Mikroträger weisen eine unregelmäßige Form und eine glatte Oberfläche auf, die sich sehr gut für das Befestigen, Ausbreiten und das Wachstum von Zellen eignet, die Mikroträger sind außerdem transparent, was die Kontrolle der Kultivierung von Zellen erleichtert.

## Beste Ausführungsvariante der Erfindung

Weitere Ziele und Vorteile der vorliegenden Erfindung werden aus der nachstehenden ausführlichen Beschreibung des Verfahrens zur Herstellung von Mikroträgern zum Kultivieren von Zellen und der zum Kultivieren von Zellen hergestellten Mikroträger ersichtlich.

Zunächst erfolgt die Herausbildung von Teilchen aus Gelatine. Gelatine in Trockenform wird bis auf Teilchen unregelmäßiger Form zerkleinert und die Teilchen mit Abmessungen, die das optimale Wachstum der Zellen an denselben gewährleisten, abgeschieden. Dann werden die hergestellten Gelatineteilchen der Behandlung mit einem Quervernetzungsmittel unterworfen, als solches verwendet man vorzugsweise Formaldehyd, beziehungsweise Dialdehyde, beispielsweise, Glutaraldehyd, Glyoxal und andere.

Infolge der Behandlung mit dem Quervernetzungsmittel entsteht ein Gelatine-Blockpolymer, das die mechanische, chemische und thermische Stabilität der Mikroträger bewirkt.

Als Ausgangsprodukt können Gelatinelösungen in Wasser, im Dimethylsulfoxid, im Dimethylformamid, in der wasserfreien Essigsäure und in anderen Lösungsmitteln von Eiweiß verwendet werden. Vorzugsweise ist die Lösung von Gelatine in Wasser zu verwenden.

Dabei wird die Gelatinelösung zwecks Herausbildung von Teilchen mit einem Quervernetzungsmittel mit anschließender Zerkleinerung des entstandenen Gelatine-Blockpolymeres bis zu Teilchen unregelmäßiger Form mit Abmessungen behandelt, die das optimale Wachstum von Zellen an denselben gewährleisten.

Die Verwendung des Formaldehyds als Quervernetzungsmittel führt dazu, daß die Quervernetzung von Gelatinemolekülen infolge der Umsetzung eines Formaldehydmoleküls vorzugsweise mit einer $\varepsilon$ -Aminogruppe von Lysin in Gelatine unter Entstehung eines Hydroxymethylderivates mit anschließender Reaktion mit einer anderen freien Aminogruppe erfolgt.

Hierdurch verläuft die Quervernetzung zweier Moleküle der Gelatine durch eine Methylengruppe, die von einem Formaldehydmolekül generiert wird, was die Transparenz des Endproduktes und seine Stabilität bewirkt.

Bei der Verwendung des Dialdehyds als Quervernetzungsmittel erfolgt die Bildung von Schiffschen Basen mit jeder der Aldehydgruppen des Dialdehyds. Zur Stabilisierung der entstehenden NaCH-Bindungen und zur Erzeugung eines farblosen Endproduktes führt man die Behandlung des hergestellten Gelatine-Blockpolymeres mit einem reduzierenden Reagens durch.

Die herausgebildeten Teilchen aus dem Gelatine-Blockpolymer unregelmäßiger Form weisen eine unebene Oberfläche auf, die zum Befestigen, Ausbreiten und Wachstum von Zellen ungünstig ist. Zwecks Verleihung einer glatten Oberfläche den Teilchen werden sie deshalb mit einem proteolytischen Ferment behandelt, als solche können Trypsin, Kollagenase und anderes mehr verwendet werden.

Nach dem Auswaschen der Teilchen führt man ihre Stabilisierung durch. Bei der Verwendung des Dialdehyds als Quervernetzungsmittel erfolgt die Stabilisierung durch wiederholte Behandlung der Teilchen mit Dialdehyd und anschließende Behandlung mit einem reduzierenden Reagens. Als reduzierendes Reagens verwendet man Stoffe, die =C=H-Bindungen und Aldehydgruppen reduzieren, beispielsweise, Borhydride der Alkalimetalle, Wasserstoffperoxid und anderes.

Bei der Verwendung des Formaldehyds als Quervernetzungsmittel erfolgt die Stabilisierung der Teilchen des Gelatine-Blockpolymers mittels ihrer wiederholten Behandlung mit Formaldehyd.

Man erhält Mikroträger, die mechanisch, chemisch und thermisch stabile Teilchen aus Gelatine-Blockpolymer unregelmäßiger Form darstellen, die Abmessungen von 75 bis 350 $\mu$m und eine Dichte von 1,03 bis 1,15 g/cm$^3$ aufweisen.

Die anzumeldenden Mikroträger gewährleisten eine effektive biospezifische Wechselwirkung mit der Oberfläche der zu kultivierenden Zellen. Aufgrund des Brechungskoeffizienten, der den herzustellenden Mikroträgern eigen ist, und ihrer Transparenz ist es möglich, eine bequeme Kontrolle des Wachstums vorzunehmen. Die glatte, unregelmäßige Form der Mikroträger gibt den Zellen die Möglichkeit, sich gut zu befestigen und an ihrer Oberfläche zu wachsen. Die Mikroträger lassen sich mit üblichen Kulturmedien nicht zerstören und können zum Kultivieren von Zellen sowohl unter Laborbedingungen als auch unter großtechnischen Bedingungen verwendet werden.

Die erfindungsgemäßen Mikroträger weisen gegenüber den im bekannten Verfahren hergestellten Mikroträgern einen höheren Reinheitsgrad auf, weil in ihnen die Beimenungen des Suspendierungsmediums (Öl, Kohlenwasserstoffe) fehlen, das im bekannten Verfahren zum Einsatz kommt, die im weiteren eine toxische Wirkung auf Zellen ausüben. Die Verbesserung der Qualität der gemäß dem angemeldeten Verfahren erzeugten Mikroträger wird auch dadurch erreicht, daß das Verfahren eine höhere Homogenität der Korngrößenzusammensetzung der Mikroträger (98% der Teilchen mit Abmessungen von 100 bis 250 $\mu$m) gewährleistet. Die gemäß dem bekannten Verfahren hergestellten Mikroträger sind weniger homogen (75 bis 80% der Teilchen mit Abmessungen von 100 bis 250 $\mu$m).

Das erfindungsgemäße Verfahren ermöglicht es außerdem, die technologische Prozeßführung infolge der Ausschließung der Stufe der Dispergierung und der mehrmaligen Auswaschungen (gemäß dem bekannten Verfahren) zu vereinfachen, sowie erlaubt es, die apparative Ausgestalung zu vereinfachen.

Zur besseren Erläuterung der vorliegenden Erfindung werden nachstehende Beispiele der Ausführung des Verfahrens zur Herstellung von Mikroträgern zum Kultivieren von Zellen und zur Prüfung der Mikroträger angeführt.

Beispiel 1

Man vermischt 400 ml 25%ige wäßrige Gelatinelösung, die bei einer Temperatur von 50°C hergestellt wurde, mit 150 ml 25%iger wäßriger Glyoxal-Lösung unter einem intensiven Vermischen bei Raumtemperatur.

Das erzeugte Gelatine-Blockpolymere wird durch ein Sieb mit einer Maschengröße von 250 $\mu$m durchgepreßt. Die er-

zeugten Teilchen (mit einem Durchmesser nicht über 250 μm) werden mit 0,25%iger Trypsinlösung in einer 0,15 molaren Phosphatpufferlösung bei einem pH-Wert 7,6, die in einer Menge von 550 g der Teilchen des Gelatine-Blockpolymeres pro 0,5 Liter Trypsinlösung genommen werden, bei Raumtemperatur unter ständigem Vermischen behandelt. Danach werden die Teilchen des Gelatine-Blockpolymeres mit 0,15 molarer NaCl-Lösung zwecks Entfernung des Trypsins und der löslichen Produkte der Fermentenspaltung des Gelatine-Blockpolymeres ausgewaschen. Den ausgewaschenen Teilchen setzt man 100 ml 25%ige Glyoxal-Lösung zu und hält man diese innerhalb von 15 Minuten bei Raumtemperatur.

Dann wird die Glyoxal-Lösung mittels Filtrierung an einem Sieb mit einer Maschengröße von 100 μm entfernt, die Teilchen werden in 1 Liter 3%iger wäßriger Wasserstoffperoxidslösung suspendiert und während 30 Minuten gehalten, wonach man die erzeugten Mikroträger mit 0,15 Mol NaCl-Lösung auswäscht.

Die in der 0,15 molaren NaCl-Lösung suspendierten Mikroträger sterilisiert man durch Behandlung im Autoklaven bei einer Temperatur von 120°C während 15 Minuten. Man erhält 500 g Mikroträger mit einer Teilchengröße von 200 bis 250 μm unregelmäßiger Form und einer Dichte von 1,07 g/cm³.

Die erhaltenen Mikroträger wurden bei der Züchtung von oberflächen-abhängigen Nierenzellen von Grünaffen geprüft.

Die Kultivierung erfolgte in schwebenden Flakons mit 250 ml-Inhalt, in 150 ml Iegle - Nährboden mit 5% Kalbsserum. Die Konzentration der Mikroträger betrug 8.9³ Teilchen/ml des Nährbodens. Die Ergebnisse der Kultivierung sind in nachstehender Tabelle dargestellt.

Beispiel 2

Man vermischt 200 ml 35%ige wäßrige Gelatinelösung, die bei einer Temperatur von 50°C hergestellt wurde, mit 100 ml 25%iger wäßriger Lösung des Glutaraldehyds unter intensivem Vermischen bei Raumtemperatur. Das hergestellte Gelatine-Blockpolymere wird durch ein Sieb mit einer Maschengröße von 200 μm durchgepreßt. Die hergestellten Teilchen mit einem Durchmesser nicht über 200 μm) werden mit

0,5%iger Kollagenaselösung in 0,15 molarer Phosphatpufferlösung, die in einer Menge von 270 g der Teilchen des Gelatine-Blockpolymeres pro 230 ml Kollagenase-Lösung genommen wird, bei einer Temperatur von $37 \pm 1^{o}$C unter ständigem Vermischen behandelt.

Dann werden die Teilchen mit 0,15 molarer NaCl-Lösung gewaschen. Die ausgewaschenen Teilchen behandelt man hintereinander mit 130 ml 25%iger Lösung des Glutaraldehyds bei Raumtemperatur, dann mit 0,15 molarer NaCl-Lösung zwecks Entfernung des nichtumgesetzten Glutaraldehyds und mit 0,5 l reduzierendem Reagens der 2%igen Lösung des Natriumborhydrids bei Raumtemperatur, wonach man sie reichlich mit Wasser bis zum neutralen pH-Wert der Spülflüssigkeit auswächst.

Danach werden die hergestellten Mikroträger in 0,5 l 0,15 molarer NaCl-Lösung resuspendiert, in 10 bis 15 Minuten mit einer frischen Portion einer isotonischen NaCl-Lösung ausgewaschen und durch Behandlung in einem Autoklaven bei einer Temperatur von $120^{o}$C während 15 Minuten sterilisiert. Man erhält 250 g Mikroträger mit einer Teilgröße von 150 bis 200 $\mu$m unregelmäßiger Form und einer Dichte von 1,05 g/cm$^3$.

Der erhaltene Mikroträger wurde ähnlicherweise wie in Beispiel 1 beschrieben geprüft. Die Ergebnisse der Prüfungen sind in nachstehender Tabelle angeführt.

Beispiel 3

Mikroträger werden ähnlicherweise wie in Beispiel 2 beschrieben hergestellt. Als Ausgangslösung verwendet man eine 20%ige Gelatinelösung, als proteolytisches Ferment - - 0,75%ige Chymotrypsin-Lösung und als reduzierendes Reagens - 3%ige Wasserstoffperoxidlösung. Man erhält 250 ml Mikroträger mit einer Teilchengröße von 150 bis 200 $\mu$m unregelmäßiger Form und einer Dichte von 1,06 g/cm$^3$.

Die erhaltenen Mikroträger wurden ähnlicherweise wie in Beispiel 1 beschrieben geprüft. Die Prüfergebnisse sind in nachstehender Tabelle angeführt.

Beispiel 4

Die Herstellung der Teilchen des Gelatine-Blockpoly-

- 10 -

meres erfolgt ähnlicherweise wie in Beispiel 1 beschrieben. Die hergestellten Teilchen des Gelatine-Blockpolymeres werden mit 0,75 1 0,25%iger Trypsinlösung in 0,15 molarer Phosphatpufferlösung, bei einem pH-Wert von 7,6 bei einer Temperatur von 25°C unter ständigem Vermischen behandelt. Dann behandelt man die Teilchen ähnlicherweise wie in Beispiel 1 beschrieben. Man erhält 475 g Mikroträger mit einer Teilchengröße von 200 bis 250 μm unregelmäßiger Form und einer Dichte von 1,06 g/cm³.

Die erhaltenen Mikroträger wurden ähnlicherweise wie in Beispiel 1 beschrieben geprüft. Die Ergebnisse der Prüfungen sind in nachstehender Tabelle dargestellt.

Beispiel 5

Die Herstellung der Teilchen des Gelatine-Blockpolymeres erfolgt ähnlicherweise wie in Beispiel 1 beschrieben. Die hergestellten Teilchen werden mit 1,25 1 0,75%iger Chymotrypsin-Lösung in 0,06 molarer Phosphatpufferlösung mit einem pH-Wert von 6,4 bei einer Temperatur von 20°C unter ständigem Vermischen behandelt. Dann werden die Teilchen ähnlicherweise wie in Beispiel 1 bearbeitet. Man erhält 500 g Mikroträger mit einer Teilchengröße von 200 bis 250 μm unregelmäßiger Form und einer Dichte von 1,08 g/cm³.

Die erhaltenen Mikroträger wurden ähnlicherweise wie in Beispiel 1 beschrieben geprüft. Die Ergebnisse der Prüfungen sind in nachstehender Tabelle angeführt.

Beispiel 6

200 g Trockengelatine mit Abmessungen der Trockenteilchen von 75 bis 90 μm, die mittels Durchsieben der zerkleinerten Gelatine durch Siebe erzeugt wird, behandelt man nach dem Quellen in Wasser mit 500 ml 40%iger Formaldehydlösung während 10 Minuten. Der Überschuß an Formaldehyd wäscht man mit Wasser aus, und die hergestellten Teilchen des Gelatine-Blockpolymeres behandelt man mit 0,25%iger Trypsin-Lösung in 0,15 molarer Phosphatpufferlösung mit einem pH-Wert von 7,6, die in einer Menge von 550 g Teilchen pro 0,5 1 der Trypsinlösung genommen wird, bei Raumtemperatur und unter ständigem Vermischen. Danach werden die Teilchen mit 0,15 molarer NaCl-Lösung ausgewaschen und mit 500 ml 40%iger Lösung des Formaldehyds während 10 Minuten behandelt. Die hergestellten Mikroträger filtert man ab, wäscht man mit 0,15 molarer NaCl-Lösung aus. Die in 0,15 molarer Lösung suspendierten Mikroträger sterilisiert man durch Behandlung in einem Autoklaven bei einer Temperatur von 120°C während 15 Minuten. Man erhält 550 g Mikroträger mit einer Teilchengröße von 160 bis 200 $\mu$m unregelmäßiger Form und einer Dichte von 1,15 g/cm$^3$. Die erhaltenen Mikroträger wurden ähnlicherweise wie in Beispiel 1 geprüft. Die Ergebnisse der Prüfungen sind in nachstehender Tabelle angeführt.

Beispiel 7

Man behandelt 200 g Trockengelatine mit Abmessungen der Trockenteilchen von 8 bis 100 $\mu$m, die mittels Durchsieben der zermahlenen Gelatine in einem Luftstrahl hergestellt wurde, mit 500 ml 20%iger Formaldehydlösung. Im weiteren erfolgt die Prozeßführung ähnlicherweise wie in Beispiel 1, als proteolytisches Ferment verwendet man 25%ige Chymotrypsin-Lösung, und die Stabilisierung der Teilchen führt man mit 20%iger Lösung des Formaldehyds durch. Man erhält 590 g Mikroträger mit einer Teilchengröße von 180 bis 250 $\mu$m unregelmäßiger Form und einer Dichte von 1,02 g/cm$^3$. Die erhaltenen Mikroträger wurden ähnlicherweise wie in Beispiel 1 geprüft. Die Ergebnisse der Prüfungen sind in nachstehender Tabelle angeführt.

Beispiel 8

Man behandelt 200 g Trockengelatine mit Abmessungen der Trockenteilchen von 70 bis 90 $\mu$m, die mittels Durchsieben der zermahlenen Gelatine hergestellt wurde, mit 500 ml 30%iger Formaldehydlösung während 10 Minuten. Der Überschuß an Formaldehyd wäscht man mit Wasser aus, und die hergestellten Teilchen des Gelatine-Blockpolymeres, die in einer Menge von 550 g Teilchen pro 0,5 l Trypsin-Lösung genommen werden mit 0,5%iger Trypsin-Lösung in 0,15 molarer Phosphatpufferlösung mit einem pH-Wert von 7,6, bei Raumtemperatur und unter ständigem Vermischen behandelt. Danach wäscht man die genannten Teilchen mit 0,15 molarer NaCl-Lösung aus und behandelt mit 500 ml 30%iger Formal-

dehydlösung. Die hergestellten Mikroträger filtert man ab, wäscht man mit 0,15 molarer NaCl-Lösung aus. Die in der 0,15 molarer NaCl-Lösung suspendierten Mikroträger sterilisiert man durch Behandlung in einem Autoklaven bei einer Temperatur von 120°C während 15 Minuten. Man erhält 510 g Mikroträger mit einer Teilchengröße von 160 bis 200 $\mu$m unregelmäßiger Form und einer Dichte von 1,07 g/cm$^3$.

Die erhaltenen Mikroträger wurden ähnlicherweise wie in Beispiel 1 geprüft. Die Prüfergebnisse sind in nachstehender Tabelle angeführt.

### Beispiel 9

400 ml 25%ige wäßrige Gelatine-Lösung, die bei einer Temperatur von 50°C hergestellt wurde, vermischt man mit 100 ml 35%iger wäßriger Formaldehydlösung unter intensivem Vermischen. Das hergestellte Gelatine-Blockpolymer drückt man durch ein Sieb mit einem Maschendurchmesser von 200 $\mu$m durch. Die hergestellten Teilchen (Durchmesser nicht über 200 $\mu$m) behandelt man mit 0,25%iger Trypsin-Lösung in 0,15 molarer Phosphatpufferlösung mit einem pH-Wert von 7,6 bei Raumtemperatur und ständigem Vermischen. Danach werden die Teilchen mit 0,15 molarer NaCl-Lösung zwecks Entfernung von Trypsin ausgewaschen. Den ausgewaschenen Teilchen setzt man 100 ml 35%ige Formaldehydlösung zu und hält man bei Raumtemperatur. Dann entfernt man die Formaldehydlösung mittels Filtration und wäscht man die hergestellten Mikroträger mit 0,15 molarer NaCl-Lösung suspendierten Mikroträger sterilisiert man durch Behandlung im Autoklaven bei einer Temperatur von 120°C während 15 Minuten. Man erhält 500 g Mikroträger mit einer Teilchengröße von 150 bis 200 $\mu$m unregelmäßiger Form und einer Dichte von 1,09 g/cm$^3$. Die erhaltenen Mikroträger wurden ähnlicherweise wie in Beispiel 1 geprüft. Die Prüfergebnisse sind in nachstehender Tabelle angeführt.

### Beispiel 10

200 g Trockengelatine mit Abmessungen der Trockenteilchen von 40 bis 60 $\mu$m, die mittels Durchsieben der gemahlenen Gelatine hergestellt wurde, behandelt man nach dem Quellen im Wasser mit 500 ml 25%iger Glutaraldehydlösung. Der Überschuß an Glutaraldehyd wäscht man mit Wasser aus und die hergestellten Teilchen des Gelatine-Blockpolymeres behandelt man mit 0,25%iger Trypsinlösung in 0,15 molarer Phosphatpufferlösung bei einem pH-Wert von 7,6 bei Raumtemperatur und unter ständigem Vermischen. Danach werden die genannten Teilchen mit entmineralisiertem Wasser zwecks Auswaschung von Trypsin und Entfernung der Produkte der Fermentenspaltung der Gelatine ausgewaschen. Den ausgewaschenen Teilchen setzt man 100 ml 25%ige Glutaraldehydlösung zu und läßt man sie bei Raumtemperatur stehen. Dann entfernt man die Lösung des Glutaraldehyds und wäscht man die genannten Teilchen mit Wasser aus. Die Teilchen werden in 1 Liter 1%iger Lösung des Natriumhydrogensulfits suspendiert und während 30 Minuten gehalten, wonach man die hergestellten Mikroträger mit 0,15 molarer NaCl-Lösung auswäscht. Die in der 0,15 molarer NaCl-Lösung suspendierten Mikroträger sterilisiert man durch Behandlung im Autoklaven bei einer Temperatur von 120°C während 15 Minuten. Man erhält 500 g Mikroträger mit einer Teilchengröße von 80 bis 120 $\mu$m unregelmäßiger Form und einer Dichte von 1,07 g/cm$^3$.

Die erhaltenen Mikroträger wurden ähnlicherweise wie in Beispiel 1 geprüft. Die Prüfergebnisse sind in nachstehender Tabelle angeführt.

Beispiel 11

Es wurde die Züchtung von primären Zellen der Hühnerembryonen an den gemäß Beispiel 2 hergestellten Mikroträger vorgenommen 18 ml eines konsistenten Rückstandes der Mikroträger, die eine Kulturfläche von etwa 5000 cm$^2$ gewährleisten, suspendiert man in 50 ml der Henkschen Salzlösung. In 10 Minuten wird die Henksche Salzlösung abgegossen und die Mikroträger werden in 50 ml Nährboden 199 mit 5% Kalbsserum, 0,25% Laktalbuminhydrolysat, 0,1% Galaktose, 20 mMol HEPES-Pufferlösung und mit Antibiotika suspendiert. Die hergestellte Suspension trägt man in einen Flakon von 250 ml-Inhalt mit schwebendem Rührer (Spinner) über.

Tabelle

Ergebnisse der Prüfungen der Mikroträger die
gemäß den angeführten Beispielen erhalten wurden

| lfd. Nr. | Mikroträger | Aussaatkonzentration der Zellen | Anzahl der gewachsenen Zellen | Koeffizient des Zuwachses der Anzahl der Zellen |
|---|---|---|---|---|
| 1 | 2 | 3 | 4 | 5 |
| | Mikroträger gemäß Beispiel 1 | $2.10^5$ Zellen/ml | $10.4.10^5$ Zellen/ml | 5,2 |
| | Mikroträger gemäß Beispiel 2 | –"– | $9,6.10^5$ Zellen/ml | 4,8 |
| | Mikroträger gemäß Beispiel 3 | –"– | $6,2.10^5$ Zellen/ml | 3,1 |
| | Mikroträger gemäß Beispiel 4 | $0,15.10^5$ Zellen/ml | $1,5.10^5$ Zellen/ml | 10 |
| | Mikroträger gemäß Beispiel 5 | –"– | $1,5.10^5$ Zellen/ml | 10 |
| | Mikroträger gemäß Beispiel 6 | $2.10^5$ Zellen/ml | $8,6.10^5$ Zellen/ml | 4,3 |
| | Mikroträger gemäß Beispiel 7 | –"– | $7,9.10^5$ Zellen/ml | 4,0 |
| | Mikroträger gemäß Beispiel 8 | –"– | $9,4.10^5$ Zellen/ml | 4,7 |
| | Mikroträger gemäß Beispiel 9 | –"– | $8,1.10^5$ Zellen/ml | 4,0 |
| | Mikroträger gemäß Beispiel 10 | $2.10^5$ Zellen/ml | $8,2.10^5$ Zellen/ml | 4,1 |

Man setzte der Suspension der Mikroträger 50 ml primärer Zellen der Hühnerembryonen im 9-tägigen Alter (die Zellen suspendiert man in einem Medium der obengenannten Zusammensetzung) zu und dem Flakon fügt man Nährboden bis zu einem Volumen von 100 ml zu. Dabei beträgt die Inokulationskonzentration der Zellen 0,5 Millionen Zellen/ml und die Konzentration der Mikroträger gewährleistet eine Kulturfläche von etwa 50 $cm^2$/ml.

Das Kultivieren führt man bei einer Temperatur von 37°C und unter leichtem Vermischen der Suspension der Mikroträger durch: am ersten Tag der Kultivierung mit einer Geschwindigkeit von 10 U/min, am zweiten und dritten Tag mit einer Geschwindigkeit von 20 U/min und weiterhin mit einer Geschwindigkeit von 50 bis 60 U/min. Beginnend mit dem zweiten Tag der Kultivierung man führt täglich den Austausch eines Teils des Nährbodens durch: am zweiten und dritten Tag tauscht man 50% des Nährbodens und weiter 70% des Nährbodens aus. Nach Ablauf von 5 bis 6 Tagen nach dem Beginn der Kultivierung bilden sich an den Teilchen der Mikroträger Abflußkulturen der primären Zellen der Hühnerembryonen heraus. Die Dichte der Kulturen am Ende der Kultivierung (6 Tage) erreicht (5,8± 0,3) Mio Zellen/ml, das heißt die Anzahl der primären Zellen der Hühnerembryonen vergrößert sich auf das 11fache in 6 Tagen. Die Zählung der Anzahl der Zellen, die an den Mikroträgern gewachsen sind, führt man nach folgender Methodik. Nach der Beendigung der Kultivierung entnimmt man eine Probe von 10 ml (vorher suspendiert man sorgfältig die Mikroträger). Nach dem Absetzen der Mikroträger in der Probe wird die überstehende Flüssigkeit abgegossen, und die Kulturen an den Mikroträger werden mehrmals mit 0,15 molarer Phosphatpufferlösung ausgewaschen.

Brispiel 12

Es wurde die Züchtung von sekundären Nierenzellen von Grünaffen an Mikroträgern durchgeführt, die gemäß Beispiel 2 hergestellt wurden.

Die sekundären Nierenzellen von Grünaffen führt man in einer Konzentration von $2.10^5$ Zellen/ml in die Suspension der Mikroträger ein, die man in einer Konzentration von 5,5 ml eines konsistenten Rückstandes (100 ml Nährbodens, was eine Kulturfläche von etwa 15 $cm^2$/ml gewährleistet). Man verwendet einen Nährboden folgender Zusammensetzung: maximaler Eagle - Nährboden mit 10% Kalbsserum, 0,25% Laktalbuminhydrolysat, 0,1% Galaktose, 20 mMol HEPES-Pufferlösung und Antibiotika. 350 ml Suspension der Mikroträger und Zellen im Nährboden führt man in den schwebenden Rührer (Spinner) mit einem Volumen von 1 Liter ein. Die Kultivierung erfolgt bei einer Temperatur von 37°C und leichtem Vermischen der Suspension: 10 U/min am ersten Tag, 20 U/min - am zweiten und dritten Tag der Kultivierung und weiter 50 bis 60 U/min. In 72 Stunden und in 120 Stunden nach dem Beginn der Kultivierung führt man den Austausch von 30% und von 70% des Nährbodens gegen frischen Nährboden durch. In 6 Tagen der Kultivierung beobachtet man fast an allen Teilchen der Mikroträger dichte Zellenkulturen. Zu diesem Zeitpunkt erreicht die Konzentration der Zellen 3,8 Mio Zellen/ml.

### Beispiel 13

Es wurde eine Prüfung zur langandauernden Kultivierung von Zellen an Mikroträgern durchgeführt, die gemäß Beispiel 2 hergestellt wurden.

Sekundäre Nierenzellen von Grünaffen kultiviert man gleicherweise wie in Beispiel 12 beschrieben. Nach der Herausbildung von konsistenten Kulturen trägt man die Mikroträger in Rollerflaschen von 0,5 l-Inhalt über, deren Innenwände vorher silikonisiert werden. In jede große Flasche trägt man je 175 ml Suspension der Mikroträger mit gezüchteten Kulturen ein. Die Kultivierung erfolgt bei einer Temperatur von 37°C, bei 10 U/min, im Nährboden dergleichen Zusammensetzung wie in Beispiel 12 beschrieben, jedoch mit einer auf 3% herabgesetzten Konzentration an Serum. Zweimal in der Woche werden 50% des Nährbodens gegen frischen Nährboden ausgetauscht. Jede Woche führt man die Zählung der Zellen nach der in Beispiel 11 genannten Methodik und das Mikroskopieren der genommenen Proben der Kulturen an Mikroträgern durch. Die Beobachtungen zeigen, daß die Zellen im Verlaufe von neun Wochen (Beobachtungsdauer) transparent bleiben, scharfe Konturen und die für sie typische Form beibehalten und von den Mikroträgern sich nicht schichtweise ablösen. Die Anzahl der Zellen während der ganzen Dauer der Beobachtung bleibt in Höhe von 3,6 ± 0,36 Mio Zellen/ml.

Beispiel 14

Es wurde die Züchtung von Zellen der Überimpfungslinie an Mikroträgern, die gemäß Beispiel 12 hergestellt wurden, in einem Nährboden mit gewöhnlicher und mit herabgesetzter Konzentration an Serum vorgenommen. An den Mikroträgern, die in einer Konzentration von 5,5 ml konsistentem Rückstand (100 ml des Nährbodens, Fläche gleich 15 cm$^2$/ml) genommen wurden, züchtet man Zellen der 4647-Linie, die aus Nieren eines gesunden erwachsenen Grünaffen erzeugt wurde. Die Inokulationskonzentration der Zellen der 4647-Linie beträgt $15.10^4$ Zellen/ml. Man verwendet einen Nährboden dergleichen, wie in Beispiel 12 beschrieben, Zusammensetzung, in einem Fall aber weist der Nährboden 10% Serum und im anderen Fall 1% Kalbsserum auf. In beiden Fällen setzt man den beiden Nährböden Natriumpyruvat bis zur Erzielung einer Konzentration von 10 mmol und Methylzellulose bis zur Erzielung einer Endkonzentration von 0,3% zu. Zum Vergleich werden diegleichen Zellenkulturen an der Oberfläche von Flachbondenflakons aus Glas mit 0,1 Liter-Inhalt gezüchtet, die Inokulationsdichte der Zellenkulturen in den Flakons ist diegleiche wie auch in der Kultur an den Mikroträgern mit einer Konzentration von $10.10^3$ Zellen/ml. Beim Kultivieren von Zellen in Nährböden mit gewöhnlicher und mit herabgesetzter Konzentration an Serum in üblichen Verfahren (an einer Glasoberfläche) und an Mikroträgern beträgt die Anzahl der Zellen, die an der Glasoberfläche unter Verwendung des Nährbodens mit 10% Serum gezüchtet wurden, bei allen anderen gleichen Bedingungen, 80,2% und bei der Verwendung des Nährbodens mit 1% Serum beträgt die Anzahl der Zellen, die an den Mikroträgern gezüchtet wurden, 42,2%.

Beispiel 15

Es wurde die Züchtung von Zellen der diploiden Linie an den gemäß Beispiel 1 hergestellten Mikroträgern vorgenommen.

Die diploiden Zellen der M-21-Linie, die aus dem Gewebe eines Menschenembryons erzeugt wurde, züchtet man an den Mikroträgern, die den in Beispiel 12 beschriebenen

- 18 -

ähnlich sind, die Inokulationskonzentration der Zellen beträgt in diesem Fall $3.10^5$ Zellen/ml. In 4 Tagen des Kultivierens erreicht die Anzahl der Zellen 1,8 Mio Zellen/ml, das heißt sie steigt auf das 6fache in 96 Stunden an, dabei ist ein großer Teil der Teilchen der Mikroträger mit einer Abgußzellenschicht bedeckt.

## Beispiel 16

Es wurden Zellen geerntet, die an den entwickelten Mikroträgern gezüchtet wurden.

Primäre Zellen der Hühnerembryonen züchtet man genauso wie in Beispiel 11 beschrieben. Nach dem Kultivieren entnimmt man eine Probe von 10 ml (wobei die Mikroträger vorher sorgfältig suspendiert werden), und man zählt die Zellen, wie das in Beispiel 11 beschrieben wurde. Die Zählung der Zellen zeigt, daß ihre Konzentration 5,7 Mio Zellen/ml erreicht. Der übrige Teil der Mikroträger wird nach dem Auswaschen in 50 ml 0,15 molarer Phosphatpufferlösung resuspendiert, die 0,25% Trypsin und 0,02% Ethylendiamintetraessigsäure aufweist. In 5 Minuten wird die überstehende Flüssigkeit vorsichtig abgegossen, die Mikroträger werden mit den Zellen in der übriggebliebenen Menge der Dispergierungslösung während 5 Minuten bei Raumtemperatur inkubiert. Danach setzt man der Mikroträger-Suspension eine Phosphatpufferlösung mit 0,5% Laktalbuminhydrolysat zu und man rüttelt den Flakon mehrmals auf. Die sich von den Mikroträgern schichtweise abgelösten Zellen werden gezählt. Die Anzahl der Zellen beträgt dabei 5,25 Mio Zellen/ml oder etwa 92%, bezogen auf die Anzahl der Zellenkerne.

## Industrielle Anwendbarkeit

Die anzumeldende Erfindung wird in der Medizin und im Veterinärwesen bei der Herstellung von Virusvakzinen und verschiedenen biologischaktiven Wirkstoffen, beispielsweise von hochaktiven Interferonpräparaten, Hormonen, Fermenten und anderen spezifischen Produkten Anwendung finden.

## PATENTANSPRÜCHE

1. Verfahren zur Herstellung von Mikroträgern zum Kultivieren von Zellen, das die Herausbildung von Teilchen aus Gelatine, die mit einem Quervernetzungsmittel behandelt wurde, und die Stabilisierung des hergestellten Endproduktes vorsieht, d a d u r c h  g e k e n n z e i c h n e t, daß die Herausbildung der Teilchen aus Gelatine mittels Behandlung der Gelatinelösung mit einem Quervernetzungsmittel mit anschließender Zerkleinerung des entstandenen Gelatine-Blockpolymeres bis zur Erzielung der Teilchen unregelmäßiger Form und der Abscheidung der Teilchen mit den Abmessungen erfolgt, die das optimale Wachstum der Teilchen auf denselben bewirken, beziehungsweise die Herausbildung der Teilchen aus Gelatine mittels Zerkleinerung der Trockengelatine bis zur Erzielung der Teilchen unregelmäßiger Form, der Abscheidung der Teilchen mit den Abmessungen, die das optimale Wachstum der Teilchen auf denselben bewirken, und mittels ihrer anschließenden Behandlung mit dem Quervernetzungsmittel erfolgt, und daß man nach der Herausbildung der Teilchen vor ihrer Stabilisierung die Behandlung dieser Teilchen mit einem proteolytischen Ferment zwecks Schaffung ihrer glatten Oberfläche durchführt.

2. Verfahren nach Anspruch 1, d a d u r c h  g e k e n n z e i c h n e t, daß man als Quervernetzungsmittel Dialdehyde beziehungsweise Formaldehyd verwendet.

3. Verfahren nach Anspruch 1 bzw. 2, d a d u r c h  g e k e n n z e i c h n e t, daß bei der Verwendung des Dialdehyds als Quervernetzungsmittel die Stabilisierung der herausgebildeten Teilchen mittels ihrer wiederholten Behandlung mit Dialdehyd mit anschließender Behandlung dieser Teilchen mit einem reduzierenden Reagens erfolgt.

4. Verfahren nach Anspruch 3, d a d u r c h  g e k e n n z e i c h n e t, daß man als reduzierendes Reagens Borhydrid der Alkalimetalle beziehungsweise Wasserstoffperoxid verwendet.

5. Verfahren nach Anspruch 1 bzw. 2, d a d u r c h  g e k e n n z e i c h n e t, daß bei der Verwendung des Formaldehyds als Quervernetzungsmittel die Stabilisierung der herausgebildeten Teilchen mittels ihrer wiederholten Behandlung mit Formaldehyd erfolgt.

6. Verfahren nach einem beliebigen der Ansprüche 1 bis 4, d a d u r c h   g e k e n n z e i c h n e t, daß man als proteolytisches Ferment Trypsin beziehungsweise Kollagenase verwendet.

7. Mikroträger zum Kultivieren von Zellen, die im Verfahren gemäß Anspruch 1 bis 6 hergestellt wurden, stellen Teilchen aus einem Gelatine-Blockpolymer unregelmäßiger Form, mit Abmessungen von 75 bis 350 $\mu$m und einer Dichte von 1,03 bis 1,15 g/cm$^3$ dar.

## I. CLASSIFICATION OF SUBJECT MATTER (if several classification symbols apply, indicate all) [6]

According to International Patent Classification (IPC) or to both National Classification and IPC

IPC[4] C 12 N 5/00

## II. FIELDS SEARCHED

### Minimum Documentation Searched [7]

| Classification System | Classification Symbols |
|---|---|
| IPC[4] | C 12 N 5/00, C 12 K 9/00 |

Documentation Searched other than Minimum Documentation
to the Extent that such Documents are Included in the Fields Searched [8]

## III. DOCUMENTS CONSIDERED TO BE RELEVANT [9]

| Category [*] | Citation of Document, [11] with indication, where appropriate, of the relevant passages [12] | Relevant to Claim No. [13] |
|---|---|---|
| A | FEBS LETTERS, Vol.118, No. 1, August 1980, (Elsevier / North-Holland Biomedical Press, Netherlands), Kjell NILSSON et al "Preparation of Immobilized Animal Cells", see pages 145-150 | 1-6 |
| A | Biology of the Cell, vol.46, No. 3 1982, (Societe Francaise de Microscopie Electronique, IVRY-SUR-SEINE), Angele OBRENOVITCH et al "Microcarrier culture of Fibroblastic Cells on Modified Trisacryl Beads", see pages 249-256 | 1,7 |
| A | FR, A1, 2464994, (PHARMACIA FINE CHEMICALS AB), 20 March 1981 (20.03.81) | 1,7 |
| A | EP, A2, 0101285, (KOKEN CO. LTD), 22 February 1984 (22.02.84) | 1,7 |
| A | US, A, 4373027, (Armand Berneman), 8 February 1983 (08.02.83) | 1,7 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art.

"&" document member of the same patent family

## IV. CERTIFICATION

| Date of the Actual Completion of the International Search | Date of Mailing of this International Search Report |
|---|---|
| 9 December 1986 (09.12.86) | 12 January 1987 (12.01.87) |
| International Searching Authority | Signature of Authorized Officer |
| EUROPEAN PATENT OFFICE | |